# EUROPEAN PATENT APPLICATION

(11) **EP 3 324 414 A1**
(43) Date of publication of application: **23.05.2018**
(21) Application number: 17203110.6
(22) Date of filing: 22.11.2017
(51) Int. Cl.: G16H 20/60

(54) **COMPUTER-IMPLEMENTED METHODS AND SYSTEMS FOR DETERMINING DATA ASSOCIATED WITH CAPTURED IMAGES**

(30) Priority: 22.11.2016 US 201662425410 P
(71) Applicant: FitNow, Inc., Boston, MA 02110 (US)
(72) Inventor: DICRISTINA, Paul H., Boston, MA Massachusetts 02110 (US); LOWE, Jr., Edward W., Boston, MA Massachusetts 02110 (US); PUIDOKAS, Eric B., Boston, 02110 (US); TEAGUE, Charles J., Boston, MA Massachusetts 02110 (US)
(74) Representative: Ahmad, Sheikh Shakeel

(57) **Abstract**

Computer-implemented methods, systems, and computer program products are disclosed for determining nutrition information of nutrition items for a user of a computer device. The method includes: (a) receiving a photograph of a nutrition item from the user computer device, the photograph being captured by a camera of the user computer device; (b) analyzing the photograph and extracting a set of features from the photograph using a nutrition item feature recognition engine, and determining a nutrition item type of the nutrition item based at least in part on the one or more extracted features; (c) determining a quantity, volume, or mass of the nutrition item in the photograph; (d) determining nutritional content information of the nutrition item based on the nutrition item type and the quantity, volume, or mass of the nutrition item; and (e) transmitting the nutritional content information to the user computer device for displaying to the user.

## Description

### FIELD OF THE INVENTION

This present application relates to computer implemented methods and systems for determining data associated with captured images. More particularly, though not exclusively, the present invention is directed to a method of image capture and processing in order to extract information from the image and link that information to a specific database entry in which size of the object distinguishes between different entries in the database. One area of application of this technology is in the field of determining the nutritional content of nutrition items.

### BACKGROUND

Nutrition monitoring and, more particularly, methods and systems for determining the nutritional content of food items using computer devices is a growing area of technology. One technological area of interest is image recognition used to identify the type and quantity or size of the nutritional object which is under consideration. The use of technology within this sphere presents technical challenges at least some of which are addressed by the present invention.

### BRIEF SUMMARY OF THE DISCLOSURE

In accordance with one or more embodiments, a computer-implemented method is disclosed for determining nutrition information of a nutrition item for a user of a computer device. The computer device has a camera and being capable of communicating with a computer server system over a communications network. The method comprises the steps, performed by the computer server system, of: (a) receiving a photograph of a nutrition item from the user computer device over the communications network, the photograph being captured by the camera of the user computer device; (b) analyzing the photograph and extracting a set of features from the photograph using a nutrition item feature recognition engine, and determining a nutrition item type of the nutrition item based at least in part on the one or more extracted features; (c) determining a quantity, volume, or mass of the nutrition item in the photograph; (d) determining nutritional content information of the nutrition item based on the nutrition item type and the quantity, volume, or mass of the nutrition item; and (e) transmitting the nutritional content information to the user computer device over the communications network to be displayed to the user.

In accordance with one or more further embodiments, a computer system comprises at least one processor, memory associated with the at least one processor, and a program supported in the memory for determining nutrition information of a nutrition item. The program contains a plurality of instructions which, when executed by the at least one processor, cause the at least one processor to: (a) receive a photograph of a nutrition item from a user computer device over a communications network, the photograph being captured by a camera of the user computer device; (b) analyze the photograph and extract a set of features from the photograph using a nutrition item feature recognition engine, and determine a nutrition item type of the nutrition item based at least in part on the one or more extracted features; (c) determine a quantity, volume, or mass of the nutrition item in the photograph; (d) determine nutritional content information of the nutrition item based on the nutrition item type and the quantity, volume, or mass of the nutrition item; and (e) transmit the nutritional content information to the user computer device over the communications network to be displayed to the user.

In accordance with one or more further embodiments, a computer program product residing on a non-transitory computer readable medium is disclosed. The computer program product has a plurality of instructions stored thereon for determining nutrition information of a nutrition item which, when executed by a computer processor, cause that computer processor to: (a) receive a photograph of a nutrition item from a user computer device over a communications network, the photograph being captured by a camera of the user computer device; (b) analyze the photograph and extract a set of features from the photograph using a nutrition item feature recognition engine, and determine a nutrition item type of the nutrition item based at least in part on the one or more extracted features; (c) determine a quantity, volume, or mass of the nutrition item in the photograph; (d) determine nutritional content information of the nutrition item based on the nutrition item type and the quantity, volume, or mass of the nutrition item; and (e) transmit the nutritional content information to the user computer device over the communications network to be displayed to the user.

### BRIEF DESCRIPTION OF THE DRAWINGS

FIG. 1 is a simplified diagram illustrating a representative computer network in which a computer system for determining nutrition item information in accordance with one or more embodiments may be implemented.
FIG. 2 is a flowchart illustrating an exemplary process for determining nutrition item information in accordance with one or more embodiments.
FIGS. 3-8 are exemplary screenshots from an app used by a user for determining nutrition item information in accordance with one or more embodiments.
FIG. 9 is a flowchart illustrating an alternative exemplary process for determining nutrition item information in accordance with one or more embodiments.
FIG. 10 is a flowchart illustrating an alternative exemplary process for determining nutrition item information in accordance with one or more embodiments.
FIG. 11 is a flowchart illustrating an alternative exemplary process for determining nutrition item information in accordance with one or more embodiments.
FIG. 12 is a simplified block diagram illustrating an exemplary computer system for determining nutrition item information in accordance with one or more embodiments.

### DETAILED DESCRIPTION

As used herein, the term 'nutrition item' or 'nutrition object' refers to a specific real-world object that provides nutrition to people when consumed, e.g., a single apple or a glass of orange juice.

The term 'nutrition item type' or 'nutrition object type' refers to a group of related nutrition items whose nutritional content scales generally linearly with a nutrition item's quantity, volume, or mass, e.g., all Honeycrisp apples or all Hamlin orange juice.

Various embodiments disclosed herein relate to computer-implemented methods and systems enabling users to quickly and easily determine the nutritional content of nutrition items they are contemplating consuming using a smartphone or other computer device, and to optionally maintain a log of consumed nutrition items and associated nutrition information.

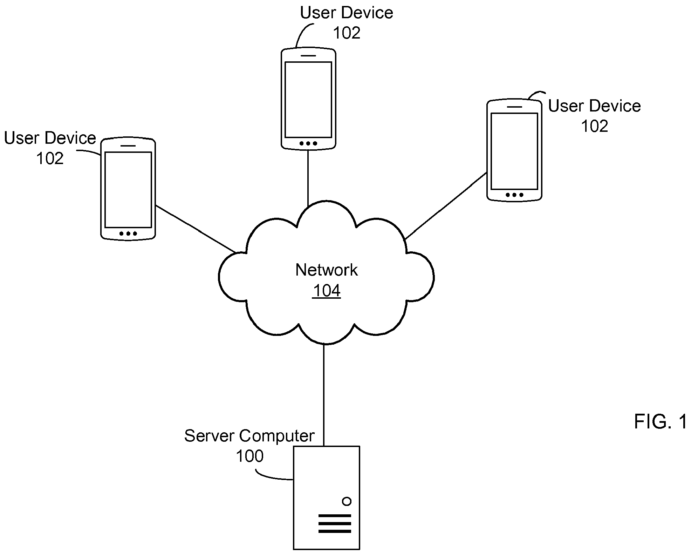

FIG. 1 illustrates an exemplary network, in which a computer system 100 for determining nutrition item information in accordance with one or more embodiments may be implemented. The system 100 is preferably implemented in a computer server system, which communicates with a plurality of client computer devices 102 operated by the users of the system 100. The client devices 102 communicate with the system 100 over a communications network 104. The communications network 104 may comprise any network or combination of networks including, without limitation, the Internet, a local area network, a wide area network, a wireless network, and a cellular network.

The client devices 102 operated by users can comprise any computing device that can communicate with the computer server system 100 including, without limitation, smartphones (e.g., the Apple iPhone and Android-based smart phones), wearable computer devices (e.g., smart watches and smart glasses), personal computers (including desktop, notebook, and tablet computers), smart TVs, cell phones, and personal digital assistants. The client devices include operating systems (e.g., Android, Apple iOS, and Windows Phone OS, among others) on which applications run. The operating systems allow programmers to create applications or apps to provide particular functionality to the devices. As discussed below, users can install an app on their client devices enabling them to quickly and conveniently determine nutritional content of nutrition items.

A representative client device 102 includes at least one computer processor and a storage medium readable by the processor for storing applications and data. The client device also includes input/output devices such as a camera, one or more speakers for acoustic output, a microphone for acoustic input, and a display for visual output, e.g., an LCD or LED display, which may have touch screen input capabilities. The client device 102 also includes a communication module or network interface to communicate with the computer server system 100 or other devices via network 104.

The system 100 uses various engines to determine nutrition item information from photos of nutrition items received from users. In accordance with one or more embodiments, the engines can include generative adversarial networks and deep convolutional neural networks leveraging residual blocks, inception modules, and region proposal layers. For example, a feature recognition engine discussed below can be pre-trained using semi-supervised generative adversarial networks. Additionally, boosted trees can also be leveraged. These methods are trained on data including manually annotated nutrition item photos, user behaviors, interaction patterns, engineered features, and user-specific data.

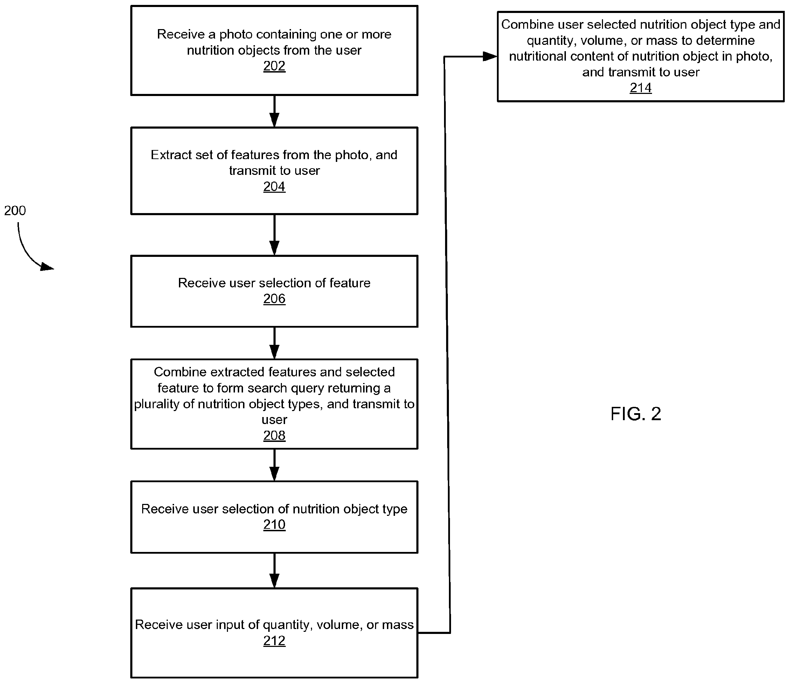

FIG. 2 is a flowchart illustrating an exemplary process 200 performed by the system 100 of determining nutrition item information in accordance with one or more embodiments.

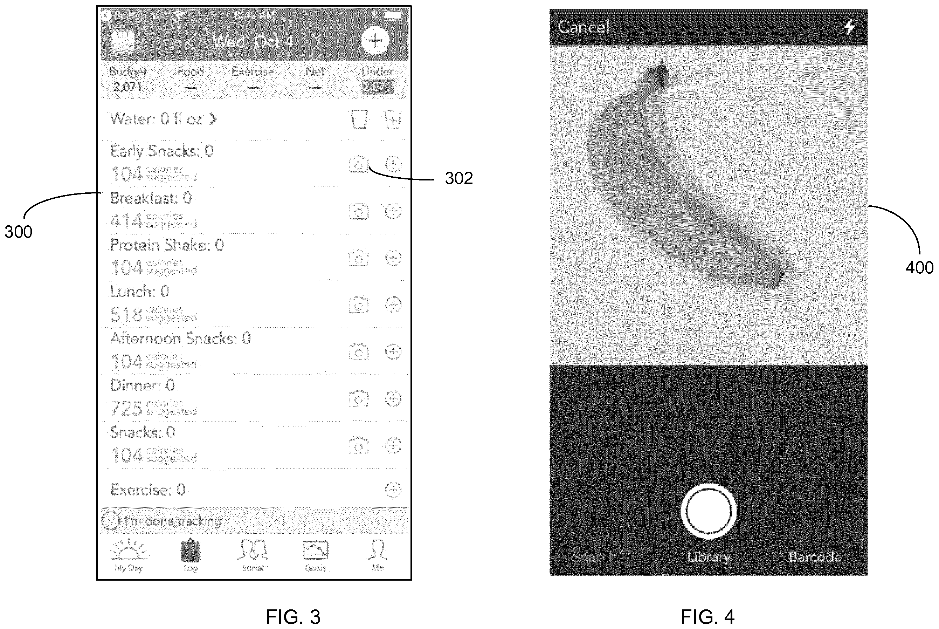

At step 202, the system 100 receives a photo containing one or more nutrition items from the user. The user can use an app downloaded on his or her smartphone or other client device to take and transmit the photo. The user can also optionally identify a meal associated with the nutrition item as shown in FIG. 3, an exemplary screenshot 300 from the app enabling the user to identify a meal. For example, the meal is breakfast, and the user selects a camera icon button 302 to launch the phone's camera app to take the photo. FIG. 4 is an exemplary screenshot 400 from the camera app, showing the user capturing a photo of a nutrition item, which in this example is a banana. The photo is sent by the app to the system 100.

At step 204, the system 100 analyzes the photo and extracts a set of suggested features from the photo using a nutrition item feature recognition engine. The feature recognition engine extracts features from the photo that are human-understandable. Features include but are not limited to commonly understood attributes (e.g., liquid, red) or higher level groupings (e.g., bread, meat).

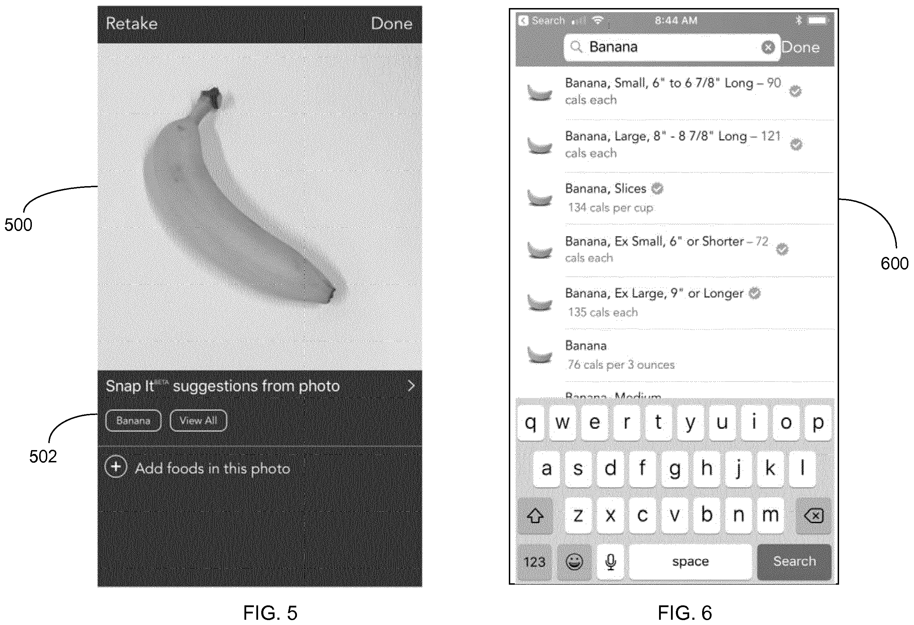

The suggested features are transmitted to the user. As shown in the screenshot 500 of FIG. 5, in this example, the suggested feature 502 displayed to the user is a banana. The user then selects a displayed feature, which is transmitted by the app to the system at step 206.

At step 208, the system combines the extracted features (from step 204) and the user selected feature (in this example, a banana from step 206) to form search query returning a plurality of nutrition item types associated with the selected feature. The nutrition item types are transmitted to user. FIG. 6 shows an exemplary screenshot 600 illustrating different possible banana products as nutrition item types.

The user selects one of the banana products, which selection is received by the system 100 at step 210.

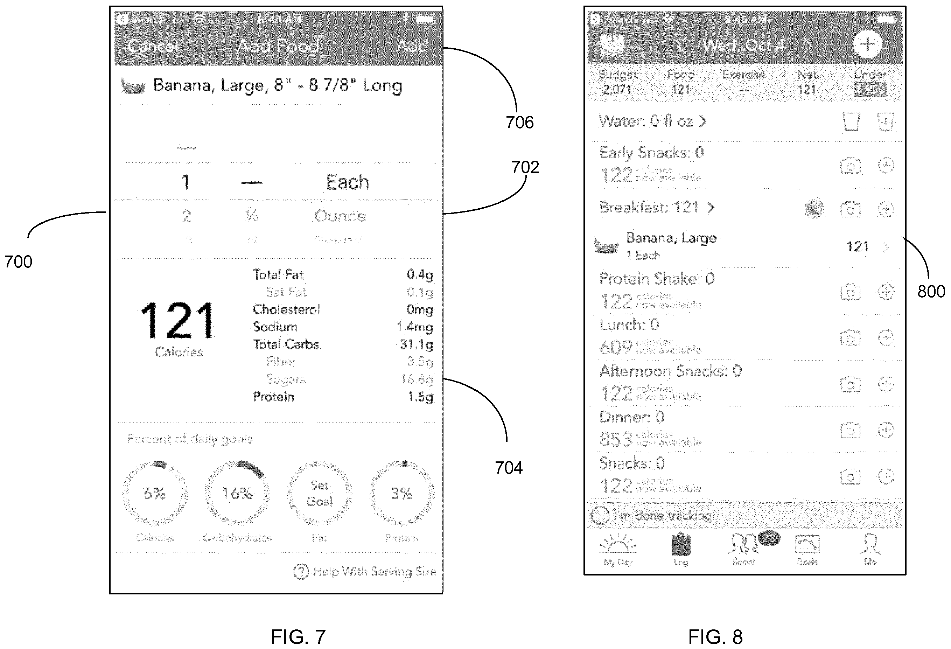

At step 212, the system receives a user input of quantity, volume, or mass of the selected raspberry product. As shown in the exemplary screenshot 700 of FIG. 7, the user can select the size of the banana serving using a virtual scroll wheel 702.

As step 214, the system 100 combines the user selected nutrition item type and quantity, volume, or mass to determine the nutritional content of a nutrition item in the photo. The app can dynamically display the nutritional content information at 704 as the user scrolls the wheel 702.

The user can select the "Add" button 706 to add the banana serving nutritional information to his or her daily nutrition log as shown in the exemplary screenshot 800 of FIG. 8.

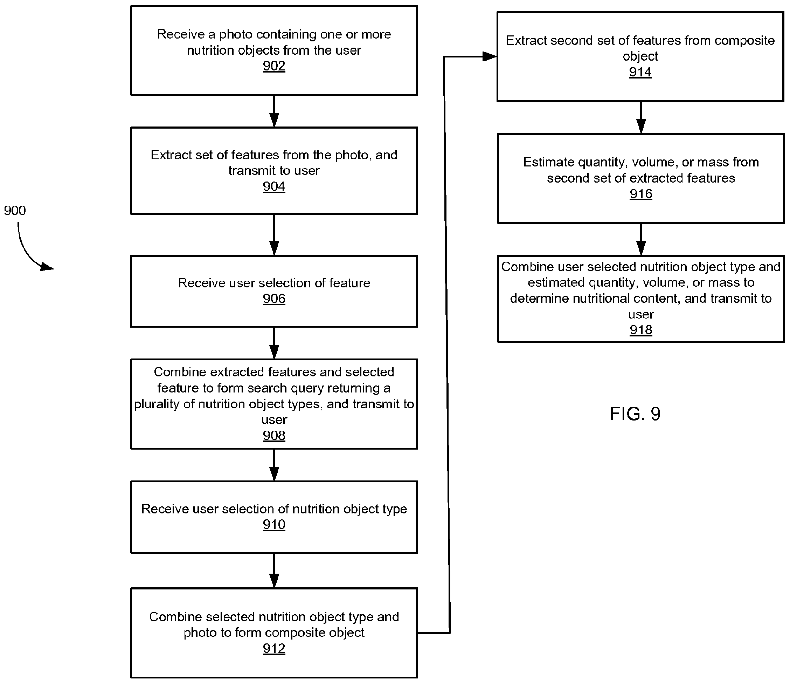

FIG. 9 is a flowchart illustrating an alternate exemplary process 900 performed by the system 100 of determining nutrition item information in accordance with one or more embodiments. The process 900 uses a quantity engine to predict the amount of the nutrition item type displayed in the photo. The prediction is returned as a quantity, volume, or mass.

Steps 902, 904, 906, 908, and 910 in FIG. 9 are generally the same as steps 202, 204, 206, 208, and 210, respectively, in FIG. 2.

At step 912, the system 100 combines the selected nutrition item type and photo to form a composite object. Composite objects are a collection of feature vectors that form the inputs for various engines. Feature vectors are defined as n-dimensional vectors of variables that numerically describe the input photo. Feature vectors can be generated through a variety of filters (e.g., deep neural network layer extraction), transformations, augmentations, and perturbations to the photo.

At step, 914 the system extracts a second set of features from the composite object. These composite objects are a collection of feature vectors that form the inputs for various engines. Feature vectors in step 914 include, but are not limited to, nutritional object segmentations, bound box estimations, voxel data which may be provided through hardware (multiple camera data), nutrition object type pairwise probabilities, and specific user probabilities.

At step 916, the system estimates a quantity, volume, or mass from the second set of extracted features.

At step 918, the system combines the user selected nutrition item type and estimated quantity, volume, or mass to determine the nutritional content of a nutrition item in the photo, and transmits the information to the user.

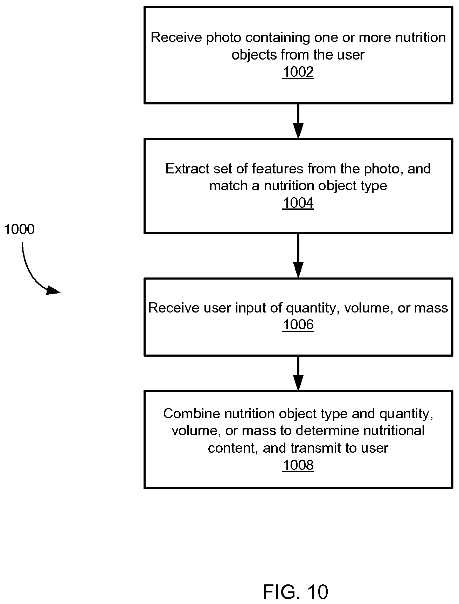

FIG. 10 is a flowchart illustrating an alternate exemplary process 1000 performed by the system 100 of determining nutrition item information in accordance with one or more embodiments.

At step 1002, the system 100 receives a photo containing one or more nutrition items from the user.

At step 1004, the system extracts a set of features from the photo and matches the pictured nutrition item to one or many specific nutrition item types. This may use other pieces of information from the user such as location or previous logging history to determine the most likely specific nutrition item types.

At step 1006, the system receives a user input of quantity, volume, or mass.

At step 1008, the system combines the nutrition item type and user-provided quantity, volume, or mass to determine the nutritional content of a nutrition item in the photo. The nutrition information is transmitted to the user.

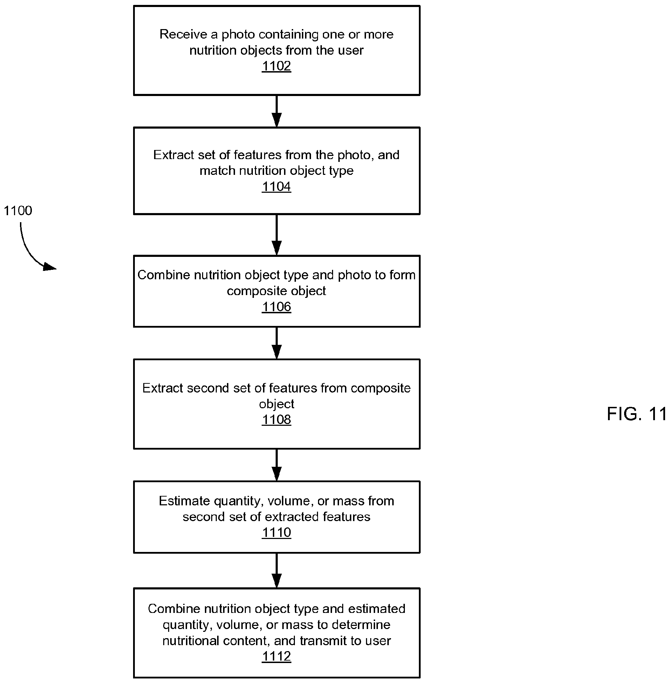

FIG. 11 is a flowchart illustrating an alternate exemplary process 1100 performed by the system 100 of determining nutrition item information in accordance with one or more embodiments.

At step 1102, the system receives a photo containing one or more nutrition items from the user.

At step 1104, the system extracts a set of features from the photo and matches a nutrition item type.

At step 1106, the system combines the nutrition item type and photo to form a composite object.

At step 1108, the system extracts a second set of features from the composite object.

At step 1110, the system estimates a quantity, volume, or mass from the second set of extracted features.

At step 1112, the system combines the nutrition item type and estimated quantity, volume, or mass to determine the nutritional content of a nutrition item in the photo. The information is transmitted to the user.

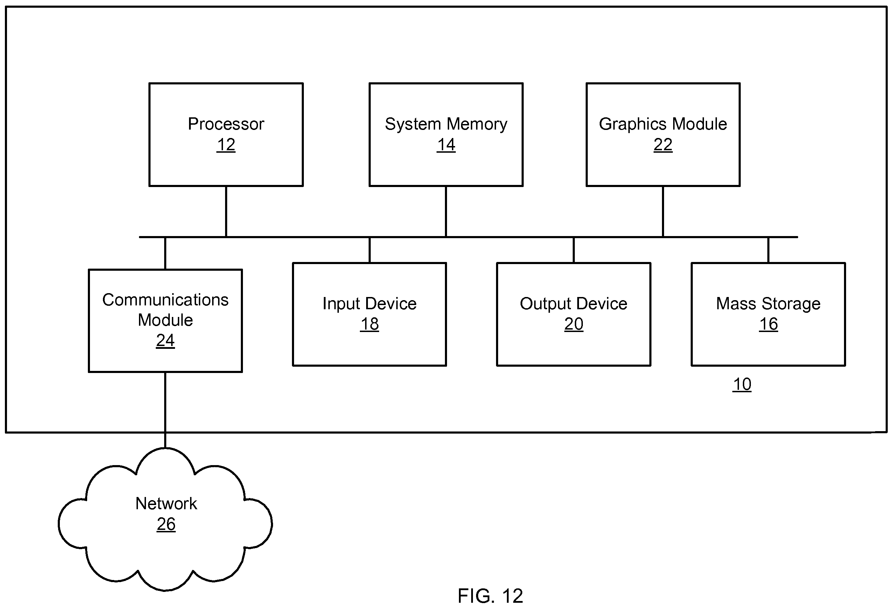

The methods, operations, modules, and systems described herein for determining nutrition item information may be implemented in one or more computer programs executing on a programmable computer system (which can be part of the server computer system 100). FIG. 12 is a simplified block diagram illustrating an exemplary computer system 10, on which the computer programs may operate as a set of computer instructions. The computer system 10 includes at least one computer processor 12, system memory 14 (including a random access memory and a read-only memory) readable by the processor 12. The computer system also includes a mass storage device 16 (e.g., a hard disk drive, a solid-state storage device, an optical disk device, etc.). The computer processor 12 is capable of processing instructions stored in the system memory or mass storage device. The computer system additionally includes input/output devices 18, 20 (e.g., a display, keyboard, pointer device, etc.), a graphics module 22 for generating graphical objects, and a communication module or network interface 24, which manages communication with other devices via telecommunications and other networks 26.

It is also to be appreciated that the data store 16 or the system memory 14 may include a database of records which provide the predetermined information relating to the nutritional object which is captured in the image. Images typically do not convey size particularly well in part because they are two-dimensional representations of three-dimensional objects. Thus, it is the matching of the image to the specific record which can be considered a technical problem particularly if the size of the object determines a different data record within the database. The present invention resolves this problem by using further data input from the user devices (smartphone for example) in a simple manner or by using automated methods for making approximations relating to size.

Each computer program can be a set of instructions or program code in a code module resident in the random access memory of the computer system. Until required by the computer system, the set of instructions may be stored in the mass storage device or on another computer system and downloaded via the Internet or other network.

Having thus described several illustrative embodiments, it is to be appreciated that various alterations, modifications, and improvements will readily occur to those skilled in the art. Such alterations, modifications, and improvements are intended to form a part of this disclosure, and are intended to be within the scope of this disclosure as determined by the claims. While some examples presented herein involve specific combinations of functions or structural elements, it should be understood that those functions and elements may be combined in other ways according to the present disclosure to accomplish the same or different objectives. In particular, acts, elements, and features discussed in connection with one embodiment are not intended to be excluded from similar or other roles in other embodiments.

Additionally, elements and components described herein may be further divided into additional components or joined together to form fewer components for performing the same functions. For example, the computer system may comprise one or more physical machines, or virtual machines running on one or more physical machines. In addition, the computer system may comprise a cluster of computers or numerous distributed computers that are connected by the Internet or another network.

Accordingly, the foregoing description and attached drawings are by way of example only, and are not intended to be limiting.

## Claims

1. A computer-implemented method of determining nutrition information of a nutrition item for a user of a computer device, said computer device having a camera and being capable of communicating with a computer server system over a communications network, the method comprising the steps, performed by the computer server system, of:
(a) receiving a photograph of a nutrition item from the user computer device over the communications network, said photograph being captured by the camera of the user computer device;
(b) analyzing the photograph and extracting a set of features from the photograph using a nutrition item feature recognition engine, and determining a nutrition item type of the nutrition item based at least in part on the one or more extracted features;
(c) determining a quantity, volume, or mass of the nutrition item in the photograph;
(d) determining nutritional content information of the nutrition item based on the nutrition item type and the quantity, volume, or mass of the nutrition item; and
(e) transmitting the nutritional content information to the user computer device over the communications network to be displayed to the user.

2. The method of Claim 1, wherein the set of features extracted from the photograph are in a human-understandable form, and wherein step (b) further comprises transmitting the set of features to the user computer device to be displayed to the user, receiving a selection of a particular feature from the user, and using the selected particular feature and the set of features to form search query returning a plurality of nutrition item types associated with the particular feature.

3. The method of Claim 2, wherein step (b) further comprises transmitting the plurality of nutrition item types to the user computer device to be displayed to the user, and receiving a user-selected single nutrition item type from the user computer device to determine the nutrition item type.

4. The method of any preceding claim, wherein step (c) comprises receiving a user-selected quantity, volume, or mass of the nutrition item type from the user computer device.

5. The method of Claim 4, wherein step (d) comprises determining the nutritional content information and transmitting the nutritional content information to the user computer device to be dynamically displayed on the user computer device as the user-selected quantity, volume, or mass changes.

6. The method of any preceding claim, wherein step (c) comprises combining the nutrition item type and the photograph to form a composite object, extracting a second set of features from the composite object, and estimating the quantity, volume, or mass of the nutrition item from the second set of features.

7. The method of any preceding claim, further comprising generating a nutrition log for the user based on the nutritional content information.

8. A computer system, comprising:
at least one processor;
memory associated with the at least one processor; and
a program supported in the memory for determining nutrition information of a nutrition item, the program containing a plurality of instructions which, when executed by the at least one processor, cause the at least one processor to:
(a) receive a photograph of a nutrition item from a user computer device over a communications network, said photograph being captured by a camera of the user computer device;
(b) analyze the photograph and extract a set of features from the photograph using a nutrition item feature recognition engine, and determine a nutrition item type of the nutrition item based at least in part on the one or more extracted features;
(c) determine a quantity, volume, or mass of the nutrition item in the photograph;
(d) determine nutritional content information of the nutrition item based on the nutrition item type and the quantity, volume, or mass of the nutrition item; and
(e) transmit the nutritional content information to the user computer device over the communications network to be displayed to the user.

9. The computer system of Claim 8, wherein the user computer device is a smartphone, a tablet computer, a wearable computer device, a personal computer, a smart TV, a cell phone, or a personal digital assistant.

10. The computer system of Claim 8 or 9, wherein the set of features extracted from the photograph are in a human-understandable form, and wherein step (b) further comprises transmitting the set of features to the user computer device to be displayed to the user, receiving a selection of a particular feature from the user, and using the selected particular feature and the set of features to form search query returning a plurality of nutrition item types associated with the particular feature.

11. The computer system of Claim 10, wherein step (b) further comprises transmitting the plurality of nutrition item types to the user computer device to be displayed to the user, and receiving a user-selected single nutrition item type from the user computer device to determine the nutrition item type.

12. The computer system of any of Claims 8 to 11, wherein step (c) comprises receiving a user-selected quantity, volume, or mass of the nutrition item type from the user computer device.

13. The computer system of claim 12, wherein step (d) comprises determining the nutritional content information and transmitting the nutritional content information to the user computer device to be dynamically displayed on the user computer device as the user-selected quantity, volume, or mass changes.

14. The computer system of any of Claims 8 to 13, wherein step (c) comprises combining the nutrition item type and the photograph to form a composite object, extracting a second set of features from the composite object, and estimating the quantity, volume, or mass of the nutrition item from the second set of features.

15. The computer system of any of Claims 8 to 14, wherein the program further comprises instructions for generating a nutrition log for the user based on the nutritional content information.
